Europäisches Patentamt

European Patent Office

Office européen des brevets

(19)

(11) Publication number: **0 646 392 A1**

(12) **EUROPEAN PATENT APPLICATION**
published in accordance with Art.
158(3) EPC

(21) Application number: **92924951.4**

(22) Date of filing: **24.11.92**

(86) International application number:
**PCT/RU92/00214**

(87) International publication number:
**WO 93/19579 (14.10.93 93/25)**

(51) Int. Cl.6: **A61N 5/00**

(30) Priority: **03.04.92 RU 5049314**

(43) Date of publication of application:
**05.04.95 Bulletin 95/14**

(84) Designated Contracting States:
**AT BE DE DK ES FR GB GR IE IT NL PT**

(71) Applicant: **KARP, Yuly Semenovich**
**ul. Kurchatova, 4-55**
**Sankt-Peterburg, 194223 (RU)**

(72) Inventor: **KARP, Yuly Semenovich**
**ul. Kurchatova, 4-55**
**Sankt-Peterburg, 194223 (RU)**

(74) Representative: **Ebbinghaus, Dieter, Dipl.-Ing.**
**et al**
**Patentanwälte v. Füner, Ebbinghaus, Finck**
**Mariahilfplatz 2 & 3**
**D-81541 München (DE)**

(54) METHOD AND DEVICE FOR INFLUENCING AN OBJECT AND APPARATUS USING SAID DEVICE.

(57) A method for influencing a object participating in a technological process consists in directing an external energy on an intermediate element which changes its functional state and influences the object participating in the technological process, and the influence is formed as a secondary radiation which is interconnected with the external energy and which is generated from the substance contained in the material of the intermediate element. The formation of the secondary radiation flow with desired parameters is effected by periodical variation of the vector of anisotropy of the substance of the intermediate element and the thus formed flow of the secondary radiation is directed on the object. The device comprises a external energy source (2) and a intermediate element (3) capable of generating a influence in the form of secondary radiation matching with the external energy from the source (2) and resulting from the periodical change of the vector of anisotropy of the substance contained in the material of the intermediate element (3). The device is used as apparatus for programmed stimulation of self-regulation of biological objects for correction of their functional state.

FIG. 5

EP 0 646 392 A1

FIELD OF TECHNICS

The invention pertains to general-purpose physical means. More accurately, the invention pertains to methods for influencing an object, devices implementing such methods, and apparatuses using such devices.

PRIOR ART

Certain methods of influencing an object participating in a process have been reported earlier. They pertain to processes of energy interaction among different materials, particularly, in chemistry, power engineering, agriculture, biology.

One such method is known from the German application "Substrate, Method, and Apparatus for Its Production" (DE, AI 3612315). This method suggests substrates' creation by means of excitation of substances from an electromagnetic energy source. The substrate acquires special energy properties (i.d. reveals an energetically active "charge"), suitable for many applications, e.g. in agriculture, and for influencing human/animal body processes.

The said substrate can be considered an intermediate unit for a method of influencing an object participating in a process, where an external energy is directed on the said intermediate unit, said intermediate unit changes its functional state and exerts an influence on, at least, one object participating in a process.

The foregoing patent application does not disclose either in what way do substrate properties change, or by what means are they transmitted further. A time diversity exists between such process components as creation of a substrate and exertion of an influence on an object A nature of the influence is not defined; an efficient control over its exertion is not available; the substrate's "active charge" may abate or dissipate with time; and an immediate, contact, invasive influence on substances and materials is required.

A device shaped in a specific way amplifies an emission. (FR, A, 2421531). It is suggested for influencing geoelectromagnetic field, gravitation, refractive index of the air, reactions of combustion, and other objects and processes.

An emission from a certain shaped form constitutes an acting factor. The said form may be considered an intermediate unit in a process where an influence is exerted on, at least, one participating in the process object The intermediate unit changes its functional state and affects participating object.

The said shaped form is connected to electric, and/or magnetic, and/or electromagnetic field generator which creates the field inside or adjacent to the form. Such generator may constitute external energy source amplifying form's emission.

However, a nature of the said emission has not yet been discovered. The only fact heretofore known is that such radiation of undefined nature renders a certain influence on objects in the proximity of emitting units. A nature of said influence on an object exposed to radiation has not been discerned; an efficient control over, and programming of required parameters of the acting factor have been unavailable.

At the present time, the described radiation does not have industrial use.

Such radiation has never been used in apparatuses for programmed stimulation of biological objects' self-regulation.

DISCLOSURE OF THE INVENTION

The invention is intended to formulate a method of influencing an object, a device implementing such method, and an apparatus using such device. By unconventional influence of an intermediate unit, said method, device, and apparatus should facilitate changes in object's structural state through synthesis of new structural links.

According to the inventions in the method of influencing an object participating in a process, where an external energy is directed on intermediate unit that changes its functional state and affects, at least, one object participating in the process, the aforementioned task is accomplished by designing an intermediate unit's influence on every object of a process as a secondary radiation (SR), said SR is interconnected with external energy directed on intermediate unit. The SR is emitted from, at least, one component substance of intermediate unit. Control over SR parameters is achieved by periodical change of anisotropy vector of, at least, one component substance of intermediate unit. The SR thus formed is directed on an object participating in process.

It is advisable to use an intermediate unit including, at least, one substance with its own anisotropy; or an intermediate unit including at least, one substance where anisotropy can be induced artificially.

2

SR parameters are subject to efficient control in accordance with a desired program. Influence exerted by intermediate unit may consist of simultaneous SR from several intermediate unit substances.

It is advisable to use, at least, one additional intermediate unit generating SR flux that is reciprocally connected to external energy source. The said SR flux is directed on a main intermediate unit which matches each additional intermediate unit's SR flux parameters with corresponding object participating in process.

At least, one intermediate unit's substance may belong to object participating in process, and control over parameters of SR flux from that intermediate unit is exercised by means of optical and/or optoelectronic elements.

It is also advisable that, at least, one substance of each intermediate unit is used to transmit SR to given direction and that object is influenced by turns of opposite in effect SR fluxes. In addition, it is recommendable that SR flux is splitted into para-phase signals directed on, at least, two zones of an object.

According to the invention, in the apparatus for influencing an object participating in process, the said apparatus comprising of an external energy source and of, at least, one intermediate unit connected to such external energy source and influencing, at least, one process object, this task is accomplished by designing each intermediate unit the way that it is capable to exert an influence in SR form, SR from every intermediate unit corresponds to an external energy from external energy source and is subject to periodical changes of anisotropy vector of, at least, one component substance of such intermediate unit.

It is proper to place a block controlling such intermediate unit's SR flux parameters on the axis of SR flux.

It is advisable that the apparatus includes a unit for programmed modification of SR flux parameters where such unit's output is connected to external energy source and/or to block controlling SR flux parameters, and such unit's input connected to object's functional state pickupdevice.

According to the invention, the task of correction of biological objects' functional state is accomplished by an apparatus for programmed stimulation of said biological objects' self-regulation embodied in the described above device for influencing an object.

The said method and device allow to implement a technological analog for natural informational interactions among substances; that is the said method and device give technical means of generation of an oriented SR flux from a substance. The said method and device allow efficient control over the said emission exercised according to desired for particular purposes program. The acting factor is equivalent to object's natural regulation signals.

In accordance to the foregoing, the invention introduces fundamentally novel process schemes permitting to intensify processes, to reduce consumption of energy, to implement novel environmentally clean options of control over processes.

Apparatuses utilizing this method are fit for broader and more efficient application in science and technique. Use of said method and device in medical and biological fields is particularly important.

BRIEF DESCRIPTION OF THE INVENTION

The invention is illustrated below by embodiment examples with references to drawings where:

Fig. 1 represents the principal scheme of apparatus for influencing an object according to the invention;

Fig. 2 - the same apparatus with two intermediate units according to the invention;

Fig. 3 - the same apparatus with SR flux parameters control block according to the invention;

Fig. 4 and 5 - the same apparatus with SR parameters programmed modification unit according to the invention;

Fig. 6 - the same apparatus with object's functional state pickup device according to the invention;

Fig. 7 - the same apparatus with several intermediate units according to the invention;

Fig. 8 - another embodiment of the apparatus according to the invention;

Fig. 9 - scheme of SR flux formation according to the invention;

Fig. 10 - one more apparatus with an external radiant energy source according to the invention;

Fig. 11 - method of directed SR flux formation according to the invention;

Fig. 12 - rotatable SR flux according to the invention;

Fig. 13 - two-phase electrostatic focusing-deflecting system used in the apparatus according to the invention;

Fig. 14 - another embodiment of the focusing-deflecting system according to the invention;

Fig. 15, 16, and 17 - schemes of SR flux parameters modification according to the invention;

Fig. 18 - one more embodiment of the apparatus according to the invention;

Fig. 19 - specific apparatus embodiment example according to the invention.

## THE BEST MODE OF CARRYING OUT THE INVENTION

A method of influencing an object participating in process consists of a sequence of the following procedures.

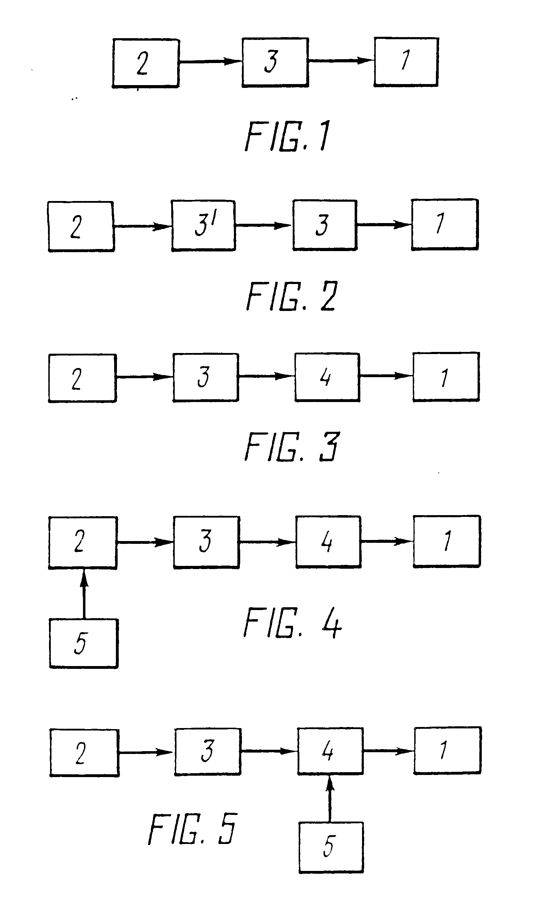

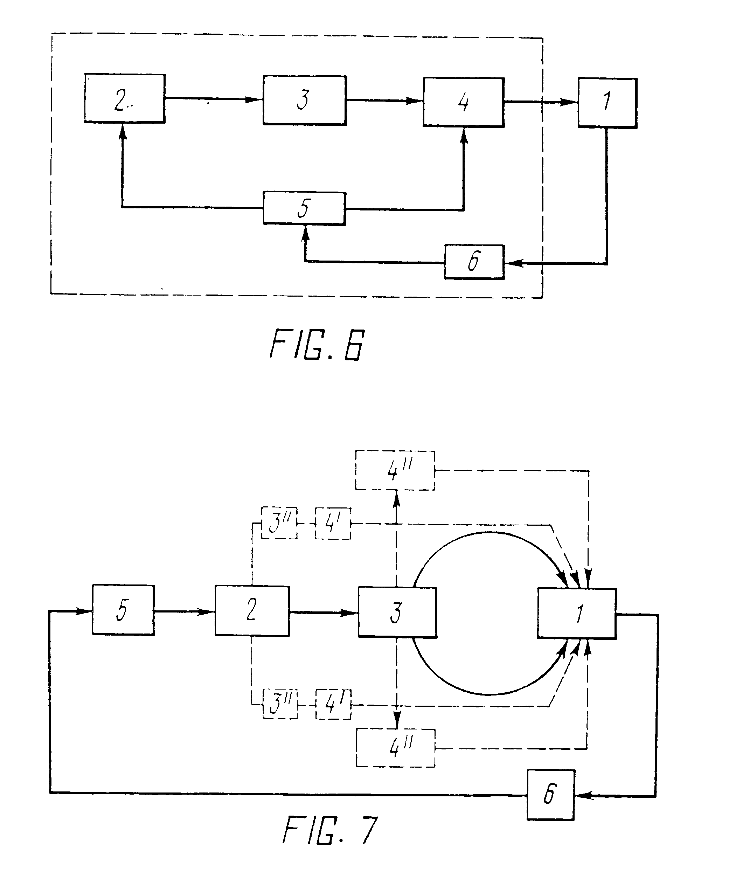

Components and/or substances participating in such processes as crystal synthesis, or catalytic reactions, or any other similar process, are designated as objects (1) of the process. (Fig. 1).

External energy from an external energy source (2) is directed through an intermediate unit (3) on the object (1). The intermediate unit (3) changes its functional state subject to the external energy and contains one or more substances that transform the external energy into SR fluxes, at least, one of which is directed on the object (1). Effectiveness of the influence is enhanced by matching substance(s) of the intermediate unit (3) with characteristics of the external energy; by matching formed SR flux with the object (1); by directivity of SR flux which is subject to its origin; by availability of immediate control over SR flux characteristics.

Described below physics processes going on in the intermediate unit (3) render SR flux formation possible.

Any substance possesses natural oscillations, in particular, at atomic and molecular levels. In certain circumstances, a substance can emit external radiation fluxes. Energy of such fluxes is composed of quantums originated from discrete binding energy changes between substance's structural elements.

For example, electrostatic or magnetic oscillations of sublattice relatively to substance's basic structure (lattice) generate quantums of energy, and thus both energy surplus and deficiency along with corresponding changes of bonds. Opposite in sign low-energy quantums mutually annihilate within their survival time. This is common for isotropic substances. In anisotropic substances, where an internal field of forces takes form of substance's own anisotropy, an oriented travel of energy quantums along anisotropy vector(s) and emanation of said energy quantums are possible. Energy quantums travel, respectively, in and against anisotropy vector's direction depending on quantums' sign. Thus, an oriented background radiation ensues.

According to the claimed method, where a substance of an intermediate unit (3) is exposed to an external energy, amplification of natural oscillations and increase in excited quantums' quantity form SR flux. Periodical variations of anisotropy vector preserve thermodynamic equilibrium. While amplification is sufficient for anisotropic substances, isotropic substances require imposition of artificial anisotropy before emanation of oriented SR fluxes occurs. Fluxes' travel direction strictly depends on substance's vector of anisotropy, both natural and artificial. Thus, here and below, where a substance emits adversely directed fluxes of energy quantums, with values exceeding those of natural background, the phenomenon is called substance's secondary radiation (SR) SR flux is capable of propagation in different mediums.

Secondary radiation fluxes travel along average anisotropy vector, in or against its direction, subject to fluxes' sign. In physical terms, fluxes are specified by, respectively, clockwise and counter-clockwise spiral rotation of energy carriers. SR flux's sign determines, for example, a nature of its interaction with fields of forces (electrostatic, magnetic), and a nature of influence exerted on an object participating in a process (stimulation or deceleration of processes).

Substance's artificial anisotropy and time variation of its anisotropy vector depend on matching of characteristics of external energy and of a substance, such as external energy's level and directivity and binding energy between substance's structural elements. A character of substance's structural links should correspond to a mode of external energy, e.g. its electromagnetic range, in order to avoid energy bypassing inside the substance.

For example, if substance's structure is formed by spin-spin interaction, then magnetic field energy is appropriate for the method of SR generation. If substance's structure is formed by dipole bonds, then electrostatic field energy is appropriate. Radiant energy can be used for deformation of crystal lattice. When SR is formed for technological purposes, external energy level is designated in order not to upset substance's thermodynamic equilibrium.

Further, formed, directed fluxes from the intermediate unit (3) exert controlled influence on an object In accordance to a desired program, methods of control of SR flux parameters have been developed. SR flux parameters are subject to (a) properties of intermediate unit (3) substances; (b) matching of the said properties with characteristics of external energy; (c) mode of external energy; (d) characteristics of external energy components responsible for creation of artificial anisotropy and deviation of its vector; (e) parameters of process parameters that have immediate effect on SR flux.

Control over SR flux is interpreted as a possibility of shaping its frequency spectrum, of focusing the beam, and of spacing the flux. A substance of the intermediate unit (3) can serve as a waveguide to transmit SR to a given direction.

Formed fluxes' mode corresponds to the object (1), particularly, to its substance. Matching is accomplished through (a) selection of an affected place, i.e. zone or point of the object (1); (b) selection of influence penetration depth, e.g. by variation of fluxes' dimensional density; (c) selection of excitation or deceleration mode of influencing the object; (d) provision for fluxes' sign variation in the area where the object (1) is located; selection of beam's geometric dimensions and intensity.

SR fluxes exert non-destructive influence with energy level adequate to that of object (1) substance's internal interactions.

As follows from above SR flux introduces a frequency-resonance code into the object (1). Frequency spectrum of a flux depends on properties of intermediate unit (3) substance, On characteristics of external energy, on and parameters of a flux control process. Such SR frequency spectrum constitutes a code synchronizing natural oscillations of substance or substances of the object (1). Everything else being the same, this produces conformal changes of molecular structures and, therefore, alters intensity of a process.

Thus, the method of influencing the object (1) comprises of the following: (a) matching external energy is directed on a substance of the intermediate unit (3) where substance's anisotropy vector periodically changes; (b) oriented SR fluxes matching to the object (1) influence the said object.

In addition, several supplemental intermediate units (3') (Fig.2) may be used along with the aforementioned principal intermediate unit (3) (Fig.1).

In order to form a SR flux, first, an external energy is directed on such substance of intermediate unit (3) properties of which match with the mode of external energy. Second, the procured oriented flux is directed on such substance of the intermediate unit (3) that matches characteristics of the flux and of the object (1).

This method of influencing the object (1) allows to use a wide variety of substances in the intermediate unit (3). This promotes the utmost matching of parameters, e.g. frequency spectrums of acting SR and substance of the object (1). Wide selection of intermediate unit substances permits to reduce their excitation energy, eliminating substance degradation under the influence of the external force, increasing its period of exploitation, and decreasing side uncontrolled radiation. Due to fluxes' directivity, the unit (3) can be placed on axis of a flux.

Substances of the intermediate unit (3) can belong to the object (1). Where the object (1) consists only of a substance of the unit (3), the SR is formed outside the object (1). Where the substance of the unit (3) is a component of the object (1), the informational flux of low-energy quantums propagates inside the object (1) in a way similar to generation of a primary flux described above. In other words, an oriented emission of low-energy quantums occurs within the substance of the object (1). Hence, formation of informational fluxes takes place inside the object (1). Zones of the object (1) can convert external energy into informational fluxes that are adequate to natural signals of process control. In this case, possible frequency spectrum discrepancies among substances of the intermediate unit (3) and of the object (1) are avoided. Substances of the object (1) resonate. The method permits to affect selected parts or structures of the object (1) in compliance to desired program.

To this end, more stringent requirements to parameters of external energy and to their matching with properties of intermediate unit substance(s) are set forth. Level, directivity, and topologic dimensions of acting factor are chosen in order to provide for desired deviation of anisotropy vector in object (1) substance's structures, and for desired effect of informational influence, rather than for unconditioned influence of the external energy used.

Focusing-deflecting flux energy control systems are used to form parameters of a SR flux. In particular, geometric, magnetic and electromagnetic optical units can focus the flux. These units are capable of control of three-dimensional shape and direction of the flux. Electrostatic optical units also permit additional energy control, that is control over wavelength and frequency of the flux.

For simplification and weight reduction, focusing units may include intermediate substance filling gap (clearance) between poles. Physical properties of such intermediate substance should correspond to a character of additional field of forces. Accordingly, substances with high magnetic permeability are used in magnetic focusing units, and substances with high dielectric constant (permittivity) are used in electrostatic focusing and controlling systems. An intermediate substance affects focusing and energy conversion of the flux.

An acting substance that matches SR frequency spectrum with properties of object (1) can be any of the following: (a) substance of supplemental intermediate unit (3'), separately or jointly with substance of main intermediate unit (3); (b) intermediate substance of additional field of forces section; or (c) part of object's (1) substance directly.

Electrostatic optical units controlling intermediate unit's SR flux parameters generate longitudinal-accelerating or longitudinal-retarding electric field. This field's gradient. determines not only focal distance, i.e. location of maximal flux density, but also wave-length of SR flux, and, hence, carrier frequency. The following dependence of change of wave-length 2 against properties of a sector of longitudinal electrostatic field where the initial wave-length is about 1 mm, has been found experimentally:

$$\lambda = \lambda_0 \, l \pm \frac{\varepsilon \, E}{\lambda_0 \, l} \, K_\lambda \qquad (1)$$

where:

"-" - corresponds to accelerating field,

"+" - corresponds to retarding field,

$\lambda_0$ - initial wavelength,

$E$ - potential applied,

$\epsilon$ - dielectric constant of intermediate unit substance,

$l$ - length of flux's path inside the dielectric,

$K_\lambda$ - coefficient, $K_\lambda = 0,7$.

Combined, simultaneous, programmed changes of flux's wave-length, dimensions, pattern, density, focus, and directivity vector's rotational speed allow to control parameters of outcoming acting SR. Besides that, intermediate unit substances can serve as means of transmission of informational SR fluxes to segments of object (1). In this case, increase in efficacy is achieved either through overlapping of processes of acting factor's transformation, matching and transmission (e.g. in one substance); or through spacing of the said processes (e.g. where different substances are used consecutively for the above purposes). These expanded options of delivery of the acting factor (informational SR flux) to the object (1), with minimal losses, substantially broaden sphere of application of the claimed method. Described heretofore parameters' matching is employed to increase method's efficacy.

Fluxes with opposite mode of influence are in turns directed on a zone of the object (1). Intensity of spectrum of natural molecular oscillations in object's substance increases. This to a greater degree stimulates synthesis of structural links, especially, in cyclic (iterative) self-regulatory systems, e.g. in cyclic chemical reactions. This also decreases threshold level of adaptation processes.

In addition to the above, para-phase external energy or SR signals are directed on, at least, two zones of the object. Application of, at least, two opposite sign signals permits to adjust the balance between these points of the object. Where the signals are sign-variable, such complex influence facilitates optimal regulation.

As follows from the above, a formed SR flux possesses the following characteristic features:

- an external energy level that is sufficient to form a SR, does not upset substance's thermodynamic equilibrium;
- the formed flux constitutes an informational flux of low-energy quantums and cannot be fixed through regular means of technical registration;
- velocity in vacuum of flux carriers is equal to the velocity of light;
- the flux alters level of organization of structural links in a detecting medium;
- the flux is oriented;
- when, at least, one vector of own or artificially induced anisotropy deviates in the transforming substance, two opposite in sign and direction fluxes spread along the average vector axis;
- a number of parameters of interaction with external fields of forces differs from previously known interactions;
- the radiation possesses high translation capacity and transmits coded information, as well as erases preceding informational action;
- control over SR topologic, spectral, power, and other parameters is available;
- transmission of a flux to an object of influence with minimal losses is available.

Listed above qualitative properties of the formed flux lead to a conclusion about its uniqueness. A wide range of devices for different industrial spheres can be designed on the basis of the claimed method.

These devices form SR fluxes which influence a substance of an object. Formed fluxes are characterized by directivity and axis. Controlling units can be placed on the said axis producing a wide range parameters of a desired influence.

An example of embodiment of the method described follows.

A semiconductor emitting diode with the radiation wave-length of 0.94 - 0.96 mm and power of 10 mW was used a source of infa-red radiation.

Supersaturated hydrothermal solution of alum was placed in a glass 3 liter flask filling it up to one third of its volume.

Radiation from the source was directed on a supplemental intermediate unit which constituted an artificially grown fluorite crystal doped with europium, where said crystal's growth axis was oriented contrary to the direction of the external energy flux from the source.

The main intermediate unit was placed behind the supplemental intermediate unit. The main intermediate unit constituted an alum crystal which primary growth axis was oriented contrary to the direction of a negative SR flux from the supplemental intermediate unit. The SR from the main intermediate unit was directed on the said alum solution continuously in the course of 24 hours.

The aggregate weight of the grown crystal was 30% more that the weight of a control crystal.

According to the invention, the device for implementation of the above method of influencing an object (1) (Fig. 1) contains an external energy source (2) and an intermediate unit (3).

Subject to type of an affected process, different classes of devices implementing the present method utilize different types of energy sources, e.g. infra-red, magnetic, electrostatic, electromagnetic, laser etc.

The intermediate unit (3) is connected to the source (2) and have the capacity of exerting an influence in the SR form, where the SR matches with the external energy from the source (2) and constitutes the radiation described above.

The device can contain two and more intermediate units (3), (3') [Fig.2], where the unit (3') is interconnected with the source (2), and the unit (3) is interconnected with the unit (3'). The SR flux from the unit (3) is directed on the object (1).

Objects with different geometric, chemical, and biological properties and containing both anisotropic and isotropic, diamagnetic and paramagnetic substances (solid, liquid, and gaseous) can serve as intermediate units (3').

Below, an intermediate unit (3') is referred to as transforming intermediate unit (3')", and an intermediate unit (3) is referred to as "working intermediate unit (3)".

SR flux's parameters control block (4) [Fig 3] is placed on the axis of the SR flux from the unit (3).

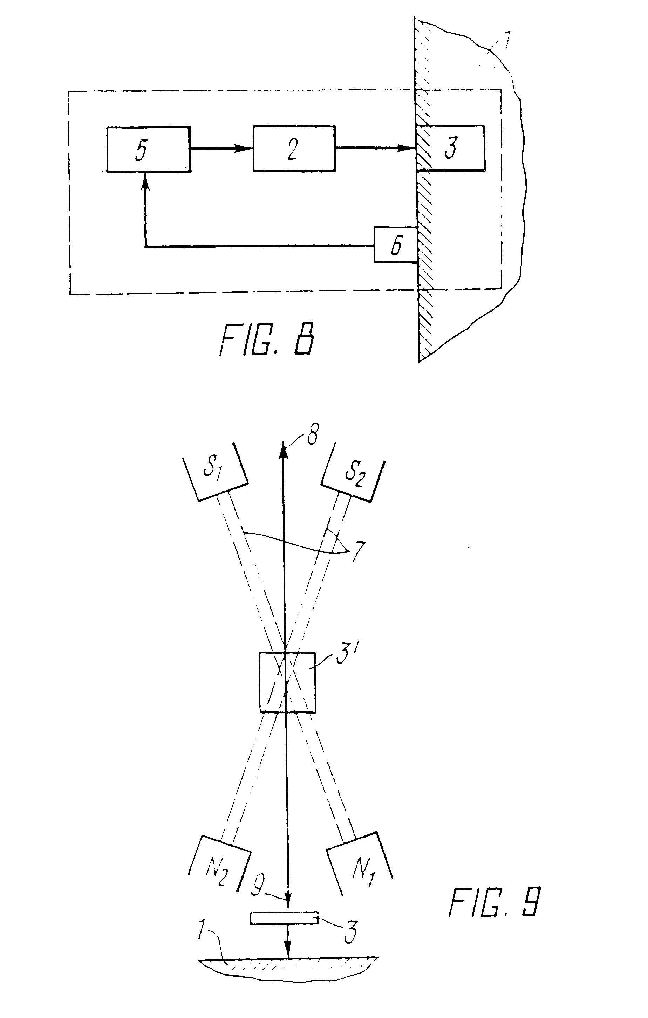

If the device employs different external energy sources, different intermediate units corresponding to the said energy sources, and different modes of influencing the object (1), multiple combinations of these elements call for a block for programmed control over parameters of SR flux (5) [Fig. 4]. The block (5) designates the following: (a) flux's divergence angle subject to transforming intermediate unit's (3') and external energy's characteristics; (b) deviation of substance's vector of anisotropy, e.g. within a plane or a solid angle, and accordingly, dimensions and frequencies of conoid rotation of the radiation vector. Practice has revealed that such topological dynamics provides for informational meaningfulness. For example, a beam with double helix three-dimensional trajectory is superposed on a frequency spectrum of a characteristic radiation of the following low-frequency modulations:

(a) those equal in frequency to a proton spins' free precession in the geomagnetic field, designated, for example, by deviating energy components of the source (2);

(b) specific interactions depending on a substance of an object, where amplitude, i.e. a diameter of a flux cone when it meets with the object (1), is subject to a treated area of the object (1).

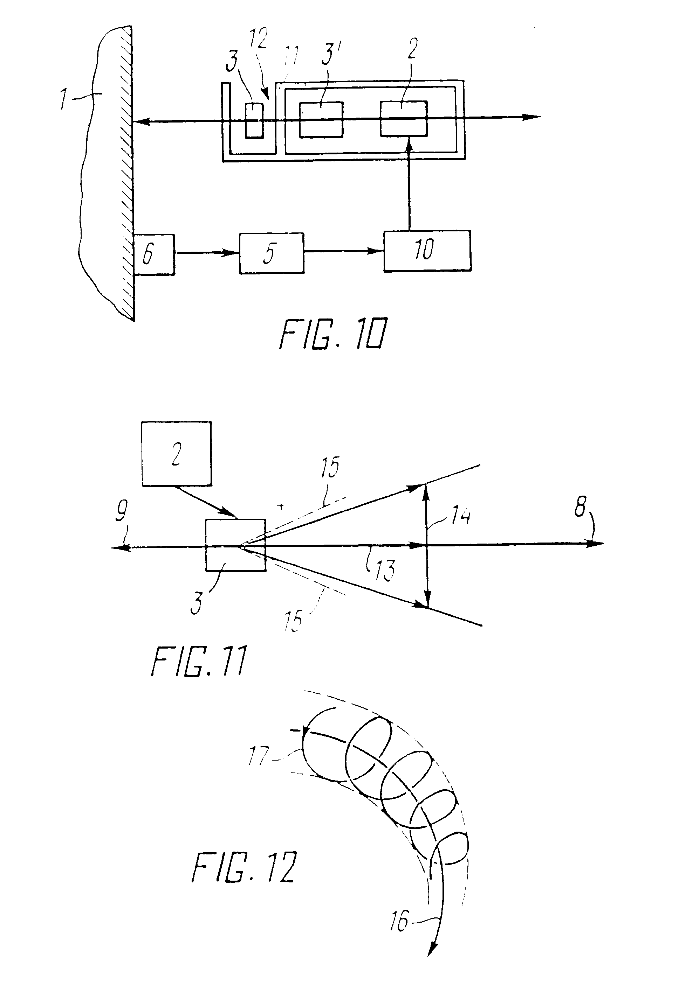

An output of the block (5) is connected either with the external energy source (2) [Fig 4], or with the controlling block (4) [Fig. 5], or simultaneously with the source (2) and the block (4) [Fig. 6].

A wide range of different modes of influence is achieved by preliminary development of programs for both external energy sources (2) and controlling block (4), subject to properties of the object (1) and to a desired impact.

Clearly, not only initial, but also current information about the object's (1) state while in process becomes very important for a proper influence selection. To this end, the device contains object's (1) functional state pickup (6) connected to the object (1) and to the input of the block (5).

Fig 7 illustrates a principal scheme of a device where para-phase signals are generated either directly by external energy source (2) and enter duplicate intermediate units (3"), said units connected to their own controlling blocks (4'); or within the substance of the intermediate unit (3) permitting generation of symmetric undistorted direct and reversed SR fluxes, where these fluxes enter two controlling blocks (4").

Where a substance of the intermediate unit (3) belongs to the object (1) [Fig. 8], the device has less complicated design.

Where an external energy source (2) generates a pulsating-direction field [Fig. 9], an intermediate unit (3') is affected, for example, by magnetic field (7) with pulsating magnetization vector ($S_1 N_1$ - $S_2 N_2$). Under this influence, SR fluxes flow from the transforming unit (3') in north (8) and south (9) pole directions. When influencing the object (1) the SR fluxes are first directed on the working unit (3). An analogous scheme takes place when an electrostatic field is used.

A radiant energy can be used for formation of a SR flux. In this case, an external energy sources (2) can be, for example, an incandescent lamps, lasers, infra-red radiation sources, and other analogous sources. For example, a semiconductor emitting diode or group of diodes [Fig. 10] connected to a power unit (10) can serve as an external energy source (2). The power unit (10) can supply, at least, two devices influencing the object (1). The external energy sources (2) and the transforming unit (3') are permanently enclosed in a case (11). The working unit (3) is designed as an easily replaceable nozzle, fastened in a cavity (12) of the case (11).

Fig. 11 illustrates SR fluxes formation principle where an intermediate unit (3) is influenced, for example, by magnetic fields from external energy source (2). Longitudinal and transversal components of the fields generated by the external energy source (2) cause deviation of anisotropy vector of the intermediate unit (3) and, hence variations of SR flux orientation. Vector (13) of directivity of longitudinal polarizing field defines vectors (8) and (9) of directivity of positive and negative SR fluxes. Where vector (13) changes its direction, the SR fluxes described by vectors (8) and (9) change their sign to the opposite. As device's and object's relative position is fixed, character of the influence changes to the opposite.

Vector (14) of the transversal magnetic field is perpendicular to the polarization vector. A change of vector's (14) direction to the opposite, according to a designated influence program, can contribute to deviation A vector of the resulting magnetic field has a three-dimensional orientation and lies within a plane or a solid angle. Vectors of SR fluxes, accordingly, lie within analogous plane or solid angles, for example, within boundaries (15).

A transversal deflecting magnetic field generated by a system of three or more poles can produce a rotatable magnetic field and therefore, conoid rotation of a SR flux where, for example, a carrier frequency (17) is superposed on a modulating frequency (16) [Fig. 12].

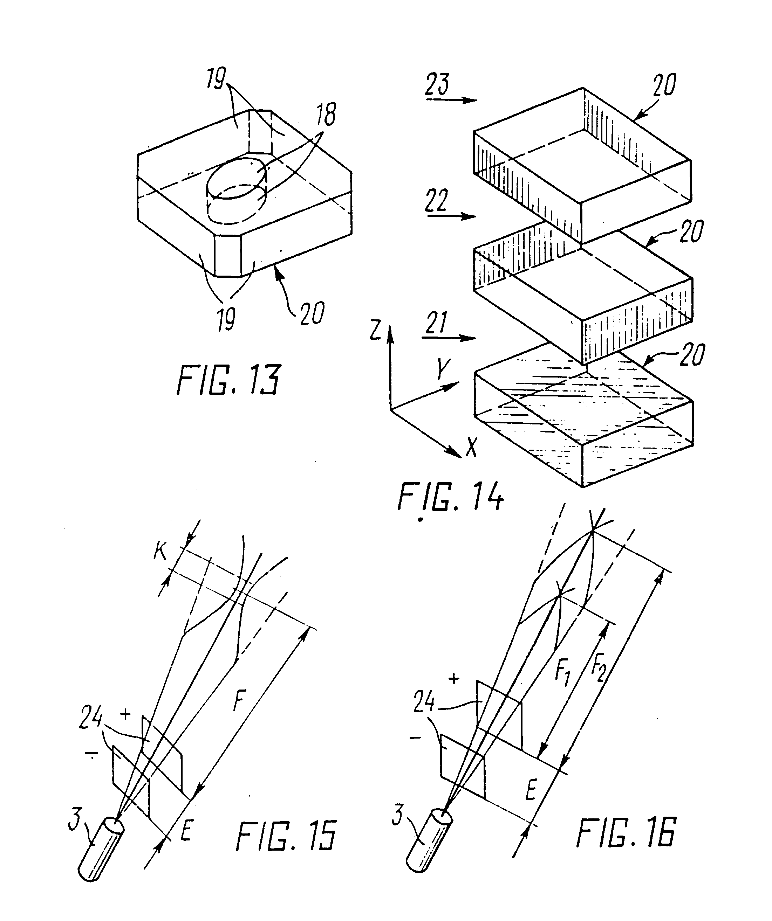

Fig. 13 represents two-phase electrostatic focusing-deflecting system with shared working gap filled by a material with high dielectric permittivity. The system contains focusing electrodes (18) and deflecting electrodes (19) embodied in a current-conducting coating on the case (20). As a variance the system can be designed with separate working gaps, including focusing gap (21) and "X" and "Y" coordinates deflecting gaps (22) and (23). [Fig. 14]. An analogous scheme takes place for magnetic and electromagnetic systems with working gaps filled with materials with high magnetic permeability. A number of deflecting phases, dimensions and properties of working gaps vary depending on a purpose of the influence and technological rationality of the construction.

Different alternatives of transformation of a formed SR flux can be suggested on an electrostatic focusing-deflecting systems' basis For examples an electrostatic system (24) [Fig. 15] is used to obtain a focused flux with a definite flux density distribution along radiation axis. Such system's parameters as air gap or filling it material, orientation of polarity, value of applied voltage, and others define focusing distance and maximal flux density sector "K".

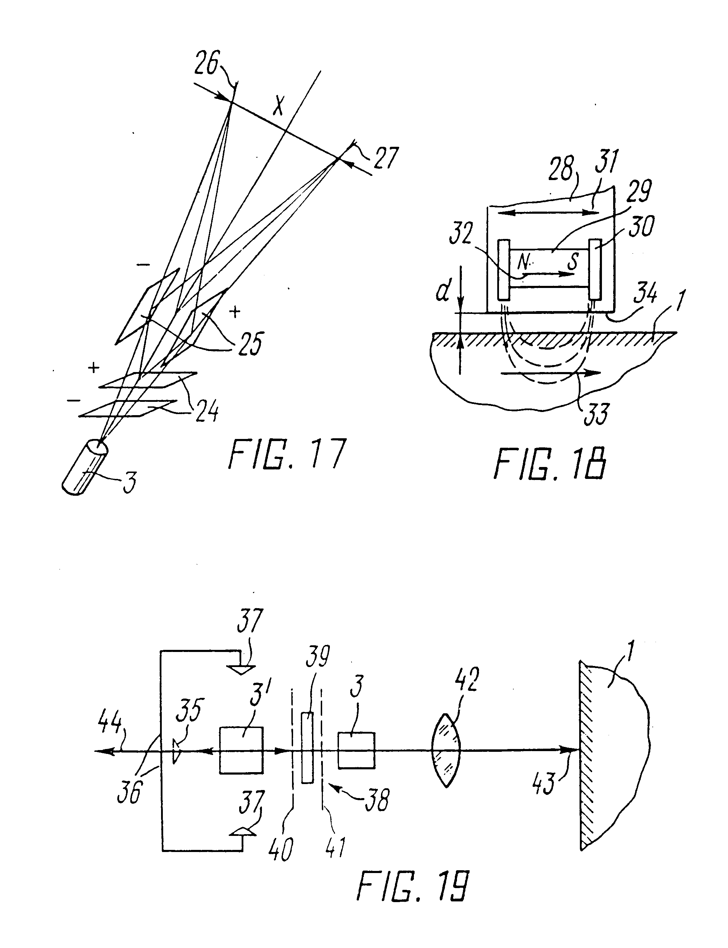

A choice of electrostatic system (24) parameters allows for precise focusing at a distance $F_1$ [Fig. 16], $F_2$ from the system (24). In order to obtain, at least, two separate focused fixes, the system (24) should contain additional focusing system (25) [Fig. 17]. Matching of systems' (24) and (25) parameters makes possible to obtain two focused in a distance X from each other fluxes (26), (27).

An electrostatic controlling system with plates (electrodes) positioned perpendicularly to an axis of a formed flux, makes it possible to control the flux's energy, particularly its wave-length. Subject to plates' sign charge relatively to passing through them flux ,5 sign, the system will render accelerating or retarding influence.

In optical systems such as geometrical optics systems, controlling units that focus or decompose the flux can be embodied in lenses or, for example, in optical elements with interfering coating (diffraction grating). It is evident that combined or separate use of magnetic, electrostatic, and geometrical optical elements will considerably broaden possibilities of formation of fluxes with different desired configuration.

Where intermediate units (3), (3') are part of the object (1), a whole new class of devices becomes possible. Such device, in particular, contains in its case a nozzle (28) [Fig. 18] that can be replaced efficiently. A permanent magnet (29) with pole concentrators (30) placed in the nozzle (28) constitutes an external energy source Magnet's (29) movement (arrow 31) is oriented along both direction of its vector (32) and relevant structures (33) of the object (1). Evidently, at the chosen distance "d" between working surface (34) of the nozzle (28) and influenced zone of the object (1), external magnetic field energy influences processes proceeding in the object (1).

Thus, a periodic permanently directed magnetic field is applied to relevant zones and points (33) of the influenced sector. A sharp cut-off (intensity gradient) travels with a chosen frequency. This provides for a contrast influence where an intermediate substance (3) as a part of the object (1) transforms the acting magnetic field into a flux of matching with the object (1) signals.

Fig. 19 represents one more embodiment variance of the device. It contains a polarizing magnet (35) with ensuing magnetic field vector and magnetic circuits (36) with pole-pieces and windings (37). The device constitutes a sign-reversible magnetic deviation system for deviation of a vector of stimulating magnetic field. In order to influence the object (1), the device also contains a controlling block (4) with longitudinal electrostatic field cells (38) filled with dielectric (39). The controlling block (4) also contains electrodes (40), (41) and lens (42) that focuses a flux of particles. Resulting opposite in sign SR fluxes (43) and (44), if necessary, can correspond to desired parameters and chosen zone of the object (1).

The device described above has proved to be a very effectual apparatus for a programmed stimulation of biological objects' self-regulation with a purpose of correction of their functional state. For example, a device embodied in accordance with Fig 1-6 can serve as a radiators particularly in reflexotherapy, for influencing significant reflex zones and points such as acupuncture points. The said radiator's influence is energetically non-destructive, distant-acting, and does not have topologic boundaries for its application. The radiator provides, if necessary, for reusable medicines serving as intermediate units. The radiator renders ecologically clean influence.

Where the device with two equivalent outlets generates two opposite in sign and direction fluxes [Fig. 7], it can serve as a basis for apparatuses that form para-phase signals directed on two or more zones of an object. The said apparatuses provide for regime of automatic self-regulation in human and animal bodies. This permits to lessen or exclude preliminary and follow-up diagnostic procedures.

Where an intermediate unit substance belongs to an object, a whole category of devices, for example, vibro-massagers [Fig. 18] becomes possible. Such device has undergone clinical testing on 12 operators. The experiments were once-only and of the same type and included preliminary psychologic testing and functional state evaluation of psychomotor and cardio-vascular indices.

Subjective appraisals of apparatuses' influence were markedly positive. The main responses were: "pleasant", "calms down", and "relaxes".

The claimed invention's most prominent use lies in the medical field. Effectiveness of medicinal preparations is enhanced by introduction of therapeutic information in the form of radiation from excited medicine without physical inculcation the preparation itself in patient's tissues. Conducted tests proved high effectiveness of influence for elimination of functional disorders of neurological origin in humans, same for animals (e.g. for treatment of horned cattle mastitis), and for plants, e.g. for elimination of harmful impact of pesticides.

Considering the claimed method's safety and possibility of portable and stationary apparatuses utilising the said method for different purposes, widespread use of the invention in different areas can be suggested. For example, research of immunity conducted by means of the proposed influence with help to disclose many phenomena that are not sufficiently clear today, and develop, accordingly, new treatments of multiple diseases including AIDS, cancer, etc.

Revealed properties of a formed SR flux make it feasible to use it in super-precise measuring devices for various trends of science and industry.

INDUSTRIAL APPLICABILITY

The invention can be utilized in the information transmission means, for synthesis of crystals, and also in medicine and agriculture.

**Claims**

1. A method of forming of an influence on object participating in a tecnolodgy process, comprising
   means of direction of external energy on an intermediate unit (3),
   as a result said intermediate unit (3) changes its functional state and influences, at least, one object (1) participating in the process wherein;
   an influence of said intermediate unit (3) on each object (1) is formed as a secondary radiation,
   said secondary radiation interconnected with said external energy directed on the intermediate unit (3),
   and said secondary radiation is emitted from, at least, one component substance of said intermediate unit (3),
   and formation of said secondary radiation fluxes with rdesired parameters is accomplished by means of periodic variations of anisotropy vector of, at least, one said component substance of the intermediate unit (3),

and said formed secondary radiation flux is directed on said object (1) of the process.

2.  A method as set forth in claim 1, <u>wherein</u>;
    said intermediate unit contains, at least, one component substance possessing its own anisotropy.

3.  A method as set forth in claim 1, <u>wherein</u>;
    said intermediate unit contains, at least, one component substance capable of possessing artificially induced anisotropy.

4.  A method as set forth in claims 1, 2, or 3 <u>wherein</u>;
    parameters of said secondary radiation flux are efficiently changed according to a desired program.

5.  A method as set forth in claims 1, 2, or 3 <u>wherein</u>;
    said influence of the intermediate unit is formed as a simultaneous secondary radiation from several component substances of the intermediate unit.

6.  A method as set forth in claims 1, 2, or 3 <u>wherein</u>;
    at least one, additional intermediate unit emits a secondary radiation flux which is interconnected with said external energy,
    and said matching secondary radiation flux is directed on a main intermediate unit,
    and said main intermediate unit is interconnected parameters of secondary radiation flux emitted from each additional intermediate unit with corresponding object of the process.

7.  A method as set forth in claim 5 <u>wherein</u>;
    at least one, additional intermediate unit emits a secondary radiation flux is interconnected with said external energy,
    and said secondary radiation flux is directed on a main intermediate unit,
    and said main intermediate unit matches parameters of secondary radiation flux from each additional intermediate unit with corresponding object of the process.

8.  A method as set forth in claims 1, 2, or 3 <u>wherein</u>;
    at least one, said component substance of intermediate unit belongs to said object.

9.  A method as set forth in claim 5 <u>wherein</u>;
    at least one, said component substance of intermediate unit belongs to said object.

10. A method as set forth in claim 7 <u>wherein</u>;
    at least one, said component substance of intermediate unit belong to said object.

11. A method as set forth in claims 1, 2, or 3 <u>wherein</u>;
    changes of desired parameters of said secondary radiation flux from each intermediate unit are accomplished by means of optical and/or optoelectronic elements.

12. A method as set forth in claim 10 <u>wherein</u>;
    changes of desired parameters of said secondary radiation flux from each intermediate unit are accomplished by means of optical and/or optoelectronic elements.

13. A method as set forth in claims 1, 2, or 3 <u>wherein</u>;
    at least one, component substance of each intermediate unit transmits said secondary radiation to a given direction.

14. A method as set forth in claim 9 <u>wherein</u>;
    at least one, component substance of each intermediate unit transmits said secondary radiation to a given direction.

15. A method as set forth in claim 12 <u>wherein</u>;
    at least one, component substance of each intermediate unit transmits said secondary radiation to a given direction.

**16.** A method as set forth in claims 1, 2, or 3 <u>wherein</u>;
objects are influenced by means of direction of opposite in effect secondary radiation fluxes on said objects in turns.

**17.** A method as set forth in claim 14 <u>wherein</u>;
objects are influenced by means of direction of opposite in effect secondary radiation fluexs on said objects in turns.

**18.** A method as set forth in claim 15 <u>wherein</u>;
objects are influenced by means of direction of opposite in effect secondary radiation fluxes on said objects in turns.

**19.** A method as set forth in claims 1, 2, or 3 <u>wherein</u>;
said secondary radiation flux is splitted into para-phase signals,
and said para-phase signals are directed on, at least, two zones of the object.

**20.** A method as set forth in claim 17 <u>wherein</u>;
said secondary radiation flux is splitted into para-phase signals,
and said para-phase signals are directed on, at least, two zones of the object.

**21.** A method as set forth in claim 18 <u>wherein</u>;
said secondary radiation flux is splitted into para-phase signals,
and said para-phase signals are directed on, at least, two zones of the object.

**22.** A device for forming of influence an object participating in a process comprising;
an external energy source (2),
at least one, intermediate unit (3) connected with said external energy source (2), and influencing,
at least one, object (1) of the process <u>wherein</u>;
each intermediate unit (3) is designed as means of influence exerted in secondary radiation form,
and said secondary radiation matches with an external energy from said external energy source (2),
and said secondary radiation is caused by periodical changes of anisotropy vector of at least, one component substance of said intermediate unit (3).

**23.** A device as set forth in claim 22 <u>that contains</u>
a block (4) which controls parameters of said secondary radiation flux is positioned on the axis of a secondary radiation flux from corresponding intermediate unit (3).

**24.** A device as set forth in claim 22 <u>that contains</u>
a block (5) for programmed control over parameters of said secondary radiation flux,
and an output of said block (5) is connected to said external energy source (2).

**25.** A device as set forth in claim 22 <u>that contains</u>
a block (5) for programmed control over parameters of said secondary radiation flux,
and outputs of said block (5) are connected to said external energy source (2) and to said secondary radiation flux's parameters control block (4).

**26.** A device as set forth in claim 22 <u>that contains</u>
a block (5) for programmed control over parameters of said secondary radiation flux,
and output of said block (5) is connected to said secondary radiation flux's parameters control block (4).

**27.** A device as set forth in claims 24, 25, or 26 <u>that contains</u>
a pickup device (6) object's functional state,
and said pickup's output is connected to an input of said block (5) for programmed control over parameters of said secondary radiation flux.

28. An apparatus for programmed stimulation of self-regulation in biological systems for purposes of correction of said biological systems' functional state,

and said apparatus constitutes a device for forming influence an object participating in a process comprising;

an external energy sources (2),

at least one, intermediate unit (3) connected with said external energy source (2) and influences, at least, on object of the process <u>wherein</u>;

design of said intermediate unit (3) allows to form an influence in secondary radiation form,

and said secondary radiation is interconnected with an external energy from said external energy source (2),

and said secondary radiation is caused by periodical changes of anisotropy vector of, at least, one component substance of said intermediate unit(3).

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19